# EUROPEAN PATENT APPLICATION

(11) **EP 1 747 753 A1**
(43) Date of publication of application: **31.01.2007**
(21) Application number: 06253950.7
(22) Date of filing: 28.07.2006
(51) Int. Cl.: A61B 5/024

(54) **A signal detection system**

(30) Priority: 29.07.2005 US 703854 P
(71) Applicant: Draeger Medical Systems, Inc., Andover, MA 01810 (US)
(72) Inventor: Sauer, Jeffrey Luke, Danver, MA 01923-1200 (US); Lemay, Charles R., Portsmouths, NH 03801-5021 (US)
(74) Representative: Vinsome, Rex Martin

(57) **Abstract**

A signal detection system for use in detecting a patient medical parameter includes a photo-detector device (30) and an amplifier (40) having an input terminal and an output terminal. A transformer (X1) has a primary winding coupled to the photo-detector device and a secondary winding coupled to the input terminal of the amplifier.

## Description

### CROSS-REFERENCED TO RELATED APPLICATIONS

This is a non-provisional application of U.S. Provisional Application Serial No. 60/703.854 filed July 29, 2005.

### FIELD OF THE INVENTION

The present invention relates to a signal detection system, and in particular to a signal detection system for use in a pulse oximeter patient monitor.

### BACKGROUND OF THE INVENTION

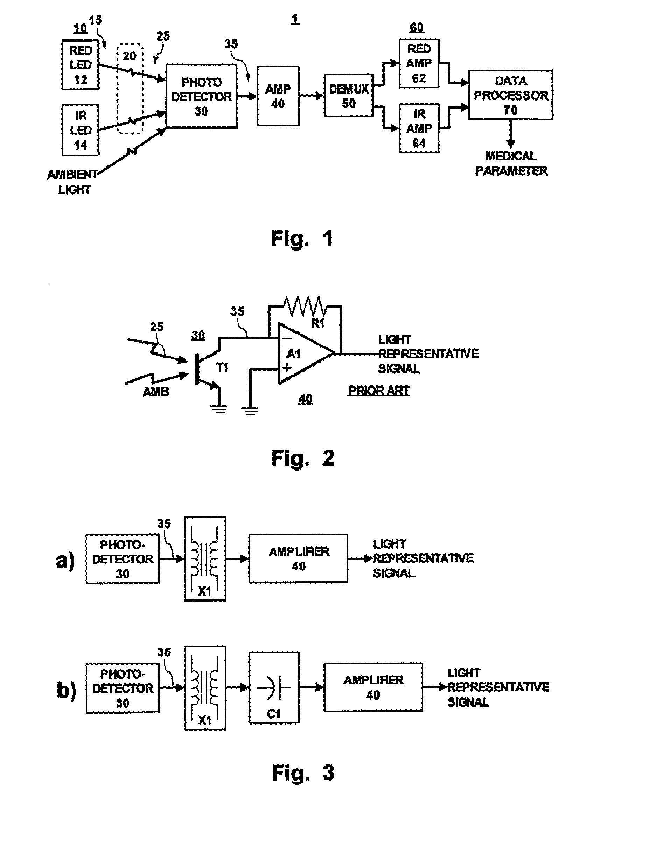

Fig. 1 is a block diagram of a portion of a pulse oximeter patient monitor device 1 in which the present invention may be implemented. In Fig. 1, a plurality 10 of light emitting devices 12 and 14 produce light signals 15. Typically, the plurality 10 of light emitting devices 12 and 14 are light emitting diodes (LEDs). The light signals 15 pass through, or are reflected off of, a blood perfused portion 20 of a patient, such as a finger, earlobe, or, in the case of a neonate, a heel of a foot. Light signals 25, resulting after passing through or being reflected off of the patient 20, are received by a photo-detector device 30. Typically, the photo-detector device 30 is a phototransistor or a photodiode. Although a single photo-detector device 30 is illustrated in Fig. 1, one skilled in the art understands that a plurality of photo-detector devices, respectively corresponding to the plurality 10 of light emitting devices 12 and 14, may be used instead.

An output terminal of the photo-detector device 30 is coupled to an input terminal of an input amplifier 40. An output terminal of the input amplifier 40 is coupled to an input terminal of a demultiplexer 50. Respective output terminals of the demultiplexer 50 are coupled to corresponding input terminals of a plurality 60 of amplifiers 62 and 64, respectively corresponding to the plurality 10 of light emitting devices 12 and 14. Respective output terminals of the plurality 60 of amplifiers 62 and 64 are coupled to corresponding input terminals of a data processor 70. An output terminal of the data processor 70 produces a signal representing a patient medical parameter. In the illustrated embodiment, the medical parameter is a blood oxygen saturation representative parameter such as an SpO₂ percentage and/or a pulse rate.

A processor, as used herein, operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. An executable application, as used herein, comprises code or machine readable instructions for conditioning the processor to implement predetermined functions, such as those of an operating system, signal detector system or other information processing system, for example, in response to user command or input.

In operation, the LEDs 12 and 14 emit light at respectively different wavelengths. Typically, one LED, e.g. 12, emits red light and the other LED, e.g. 14, emits infrared (IR) light, These light signals are multiplexed such that one LED (e.g. 12 or 14) is on at a time. The photo-detector device 30 converts the received light signals 25 into an electrical signal 35 representing the intensity of the received light signals 25. The input amplifier 40 amplifies the light representative signal 25 so that it may be processed by the subsequent circuitry. The demultiplexer 50 operates to supply the amplified light representative signal from the input amplifier 40 to the red amplifier 62 when the red LED 12 is on, and to the IR amplifier 64 when the IR LED 14 is on. The red amplifier 62 and IR amplifier 64 permit the gain of the red and IR light representative signals to be independently controlled. The data processor 70 receives the red and IR representative signals from the red and IR amplifiers 62 and 64 and processes them to produce the signal representing the patient medical blood oxygen saturation representative parameters of SpO₂ percentage and/or pulse rate.

Fig. 2 is a schematic of a prior art input amplifier 40 used in prior art pulse oximeter patient monitor devices as illustrated in Fig. 1. In Fig. 2, a phototransistor T1, corresponds to the photo-detector device 30 of Fig. 1. A collector electrode of the phototransistor T1 is coupled to an inverting input terminal of an operational amplifier (op amp) A1 and a first electrode of a feedback resistor R1. An emitter electrode of the phototransistor T1 is coupled to a source of reference potential (ground). A non-inverting input terminal of the op amp A1 is also coupled to ground. An output terminal of the op amp A1 produces a light representative signal and is coupled to a second electrode of the feedback resistor R1.

In operation, the combination of the op amp A1 and the feedback resistor R1 form a transimpedance amplifier circuit which converts the current signal from the phototransistor T1 into the light representative voltage signal. The gain of the amplifier 40 of Fig. 2 is directly proportional to the value of the feedback resistor R1. It is known that resistors produce thermal noise, termed Johnson noise. Typically, the Johnson noise is the dominant noise source in such an amplifier. The level of Johnson noise generated by a resistor increases as the square root of the value of the resistor. As described above, the gain of the amplifier 40 (of Fig. 2) is directly proportional to the value of the feedback resistor R1. Thus, increasing the value of the feedback resistor R1 increases the gain of the amplifier 40 in direct proportion to that value, while increasing the level of the Johnson noise by the square root of that value. Increasing the value of the feedback resistor R1, therefore, increases the signal-to-noise-ratio of the output signal from the amplifier 40 and decreasing the value of the feedback resistor R1 decreases the signal-to-noise ratio of the output signal from the amplifier 40.

Referring again to Fig. 1, it is also possible for ambient light to impinge on the photo-detector device 30. Ambient light sources are, for example, room lights, light through windows, and so forth. The ambient light impinges on the photo-detector 30 and is substantially constant intensity. In such a situation, the photo-detector device 30 produces a light representative signal 35 having a signal component representing the light signals 25 from the plurality 10 of LEDs 12 and 14 after passing through or being reflected off of the patient 20, and a second component representing the ambient light. Because the ambient light is substantially constant intensity, the second component, representing the ambient light, is substantially a dc offset component in the light representative signal 35. The prior art input amplifier of Fig. 2 does not remove the dc offset component in the received light signal 25. Instead, the dc component is typically removed in subsequent signal processing.

To increase the signal-to-noise-ratio, the light representative signal from the photo-detector device 30 is typically amplified with as high a gain as possible without exceeding the dynamic range of the amplifying circuits, which results in clipping of the amplified signal. However, the dc component of the light representative signal 35, caused by the ambient light, takes a portion, sometimes a substantial portion, of the dynamic range of the input amplifier 40. This requires that the gain of the amplifier be reduced to the point where the composite signal, containing the dc offset component and the LED light representative signal component, does not exceed the dynamic range of the amplifier, causing clipping of the signal. However, as described above, reducing the gain, reduces the signal-to-noise ratio.

It is desirable to provide an input amplifier which maximizes amplification without requiring a wide dynamic range, and also maximizes the signal-to-noise-ratio.

### BRIEF SUMMARY OF THE INVENTION

In accordance with principles of the present invention, a signal detection system for use in detecting a patient medical parameter Includes a photo-detector device and an amplifier having an input terminal and an output terminal. A transformer has a primary winding coupled to the photo-detector device and a secondary winding coupled to the input terminal of the amplifier.

In such a system, the transformer blocks the dc component of the light representative signal from the photo-detector device. Because only the signal component, representing the light received from the plurality of light emitting devices, after passing through or being reflected off of the patient 20, is passed through the transformer to the amplifier, the full dynamic range of the amplifier is available for the signal component The gain of the amplifier, therefore, may be increased by increasing the value of the feedback resistor R1 (Fig. 2). Further, the transformer does not introduce noise into the signal. Consequently, the signal-to-noise-ratio of the amplifier 40 is increased as well.

### BRIEF DESCRIPTION OF THE DRAWING

In the drawing:
Fig. 1 is a block diagram of a portion of a pulse oximeter patient monitor device in which the present invention may be implemented;
Fig. 2 is a schematic diagram of a prior art input amplifier which is used in prior art pulse oximeter patient monitor devices as illustrated in Fig. 1;
Fig. 3a and Fig. 3b are block diagrams of signal detection systems according to the present invention for use in detecting a patient medical parameter as illustrated in Fig. 1; and
Fig. 4 is a schematic diagram of an input amplifier according to the present invention for use in detecting a patient medical parameter as illustrated in Fig. 1.

### DETAILED DESCRIPTION OF THE INVENTION

Fig. 3a and Fig. 3b are block diagrams of signal detection systems for use In detecting a patient medical parameter. In the illustrated embodiment, the patient medical parameter is a blood oxygen saturation representative parameter such as an SpO₂ percentage and/or pulse rate. In Fig. 3a, the system includes a photo-detector device 30, an amplifier 40 having an input terminal and an output terminal, and a transformer X1 having a primary winding coupled to the photo-detector device 30 and a secondary winding coupled to the input terminal of the amplifier 40. The output terminal of the amplifier provides an amplified signal representing the light received by the photo-detector 30.

In operation, ambient light and light from the plurality 10 of light emitting devices 12 and 14 (Fig. 1), after passing through, or being reflected off of a patient 20, impinges on the photo-detector device 30. The photo-detector device 30 produces an electrical signal 35 representing the intensity of the impinging light. The electrical signal 35 from the photo-detector device 30 is supplied to the primary winding of the transformer T1. The dc offset component in the light representative signal from the photo-detector 30, produced by the ambient light, does not induce a corresponding component in the secondary winding of the transformer X1. Instead, the signal component, produced by light from the plurality 10 of light emitting devices 12, 14, alone is in the signal induced by the primary winding in the secondary winding of the transformer X1. This signal component is amplified by the amplifier 40.

As described above, because the dc offset component has been removed by the transformer X1, the full dynamic range of the amplifier 40 may be used to amplify the signal component. In addition, one skilled in the art understands that a transformer, e.g. X1, does not introduce a noise component, similar to Johnson noise, into a signal. Thus, the dc offset component is removed without introducing noise into the resulting signal component.

Fig. 3b illustrates a second embodiment of a signal detection system for use in detecting a patient medical parameter, e.g. a blood oxygen saturation representative parameter such as an SpO₂ percentage and/or pulse rate. In Fig. 3b, the system includes a photo-detector device 30, an amplifier 40 having an input terminal and an output terminal, and a transformer X1 having a primary winding coupled to the photo-detector device 30 and a secondary winding coupled to an input terminal of the amplifier 40 via a series capacitor C1.

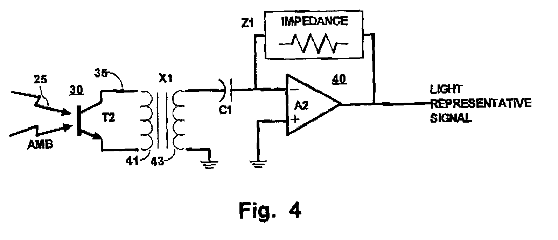

The operation of the embodiment illustrated in Fig. 3b is similar to that of the embodiment illustrated in Fig. 3a and described above. Fig. 4 is a more detailed schematic diagram of the signal detection system illustrated in Fig. 3b. In Fig. 4, the system includes a photo-detector device 30. In Fig. 4, the photo-detector device 30 is a phototransistor T2. A transformer X1 primary winding 41 is coupled in parallel with the photo-detector device 30. A collector electrode of the phototransistor T2 is coupled to one electrode of the primary winding 41 of the transformer X1, and an emitter electrode of the phototransistor T2 is coupled to the other electrode of the primary winding 41 of the transformer X1. One skilled in the art understands that other photo-detector devices may be used in place of the illustrated phototransistor. For example, a photodiode, having a cathode coupled to one electrode of the primary winding 41 of the transformer X1 and an anode coupled to the other electrode of the primary winding 41 of the transformer X1 may also be used.

The system illustrated in Fig. 4 further includes an amplifier 40, having an input and an output terminal. The amplifier 40 is an operational amplifier (op amp) A2 having an inverting input terminal acting as the input terminal of the amplifier 40, a non-inverting input terminal coupled to a source of reference potential (ground), and an output terminal acting as the output terminal of the amplifier 40. An impedance Z1 is coupled between the amplifier 40 output terminal and the input terminal. Typically the impedance Z1 is a resistance. Typically, the resistance value is around 1 megohm. The op amp A2 illustrated in Fig. 4 is configured to operate as a transimpedance, or current-to-voltage, amplifier. The output terminal of the amplifier A2 produces a voltage signal having a value representing the light received from the plurality 10 (of Fig. 1) of light emitting devices 12 and 14, after passing through or being reflected off of a patient 20.

The system further includes a transformer X1 having a primary winding 41 coupled to the photo-detector device 30 and a secondary winding 43 coupled to the amplifier 40 via a series capacitor C1. A first terminal of the transformer X1 secondary winding 43 is coupled to the input terminal of the amplifier 40 via the series capacitor C1, and the second terminal of the transformer X1 secondary winding 43 is coupled to the source of reference potential (ground). More speafically, in the illustrated embodiment, the first terminal of the transformer X1 secondary winding 43 is coupled to the inverting input terminal of the op amp A2 via the series capacitor C1.

The operation of the system illustrated in Fig. 3b and Fig. 4 is similar to that of the system illustrated in Fig. 3a. Ambient light, and light from the plurality 10 of light emitting devices 12 and 14 (Fig. 1), after passing through or being reflected off of a patient 20, impinges on the phototransistor T2 inducing a current through the collector-emitter path of the phototransistor T2. This current passes through the primary winding 41 of the transformer X1. A first component of this current is caused by the ambient light and is a substantially a dc offset current. The second component is the signal component resulting from the light signal from the plurality 10 of light emitting devices 12 and 14. Because this is not substantially a dc signal, a current corresponding to this component is induced in the secondary winding 43 of the transformer X1. This non-dc signal component current passes through the series capacitor C1 to the input terminal of the amplifier 40, i.e. to the inverting input terminal of the op amp A2. The amplifier 40 formed by the op amp A2 and the feedback impedance Z1 converts the input current into an amplified voltage signal representing the light signal from the plurality 10 of light emitting device 12 and 14.

The series capacitor C1 operates to prevent the amplifier 40 input offset voltage from being amplified along with the photo-detector 30 signal component. Practical op amps exhibit an input offset voltage. Because the resistance component of the secondary winding 43 of the transformerX1 is relatively low, typically a few ohms (e.g. 5 ohms), the amplifier offset voltage would otherwise be amplified by the amplifier gain, which is equal to the ratio of the feedback resistor divided by the resistance component of the secondary winding 43 of the transformer X1 (e.g., 1 Meg / 5 ohms = 200,000), In such a configuration, with such a high gain, a few microvolts of input offset voltage would saturate the amplifier.

Instead, the DC voltage across the series capacitor C1 is maintained equal to the offset voltage of the amplifier. This permits the voltage at the non-inverting input terminal of the op amp A2 to be maintained at ground voltage, preventing the input offset voltage from being amplified by the amplifier 40. The value of the series capacitor C1 is selected to prevent any substantial signal voltage from developing across it in response to coupling the current from the secondary winding 43 of the transformer X1 into the summing junction of the amplifier 40. Any such voltage developed across the series capacitor C1 also appears across the secondary winding 43 of the transformer X1, allowing a flux buildup in the transformer X1. This results in nonlinearity and possibly other undesirable signal degradation.

Typically, pulse oximeter patient monitors operate in the kiloHertz (kHz) range. Thus, in a typical example, a series capacitor C1 coupling a 1 microamp (µA) 2 kHz square wave signal through a capacitor of in the order of 0.47 microfarads (µF) will exhibit about 1 millivolt of ripple. One skilled in the art understands that for such a series capacitor C1, low leakage is important, but a simple ceramic, film, or other similar capacitor will generally suffice for this purpose. If some high-pass filtering is desired, the value of the series capacitor C1 may be reduced slightly, but as described above, in practice the value of the series capacitor C1 is specified to be such that at the fundamental frequency of interest (i.e. kilohertz), it prevents flux buildup in the transformer X1.

A system as illustrated in the drawing and described above advantageously employs transformer coupling in patient medical parameter signal detection and processing to remove a substantially DC signal component. The transformer advantageously maintains low impedance across a photo-detector device, such as a phototransistor or photodiode, noiselessly couples high frequency information signals to an amplifier, and rejects low frequency components that waste dynamic range within the amplifier in the system.

## Claims

1. A signal detection system for use in detecting a patient medical parameter, comprising:
a photo-detector device;
an amplifier having an input terminal and an output terminal; and
a transformer having a primary winding coupled to said photo-detector device and a secondary winding coupled to said input terminal of said amplifier.

2. The system according to claim 1 wherein said secondary winding of said transformer is coupled to said input terminal of said amplifier via a series capacitor.

3. The system according to claim 2, wherein said transformer secondary winding has a first terminal coupled to an input terminal of said amplifier via said series capacitor and a second terminal coupled to a reference potential.

4. The system according to claim 2, wherein said series capacitor prevents an amplifier offset voltage from being amplified by said amplifier.

5. The system according to claim 1, including an impedance coupled between said amplifier output terminal and said input terminal.

6. The system according to claim 5, wherein said impedance is a resistance.

7. The system according to claim 1, wherein said transformer primary winding is coupled in parallel with said photo-detector device.

8. A signal detection system for use in detecting a blood oxygen saturation representative parameter, comprising:
a photo-detector device;
an amplifier having an input terminal and an output terminal; and
a transformer having a primary winding coupled to said photo-detector device and a secondary winding coupled to an input terminal of said amplifier via a series capacitor.

9. The system according to claim 8, wherein said amplifier is an operational amplifier and said transformer secondary winding is coupled to an inverting input terminal of said operational amplifier.

10. The system according to claim 9, wherein said operational amplifier is a transimpedance amplifier.

11. A signal detection system for use in detecting a blood oxygen saturation representative parameter, comprising:
a photo-detector device;
an amplifier having an input terminal and an output terminal;
an impedance coupled between said amplifier output terminal and said input terminal; and
a transformer having a primary winding coupled to said photo-detector device and a secondary winding coupled to said input terminal of said amplifier via a series capacitor blocking transfer of a substantially DC voltage to said input terminal of said amplifier.

12. The system according to claim 11, wherein said impedance is a resistance.
